# EUROPEAN PATENT APPLICATION

(11) **EP 2 614 843 A2**
(43) Date of publication of application: **17.07.2013**
(21) Application number: 12196939.8
(22) Date of filing: 13.12.2012
(51) Int. Cl.: A61L 27/34, A61L 29/08, A61L 31/10, A61L 15/22, A61L 17/14

(54) **Methods for coating medical devices**

(30) Priority: 14.12.2011 US 201113325346
(71) Applicant: Confluent Surgical, Inc., Bedford, MA 01730 (US)
(72) Inventor: Ohri, Rachit, Framingham, MA 01701 (US); Blaskovich, Phillip, Salem, MA 01970 (US); Pham, Lan, Nashua, NH 03063 (US); Giusti, David, Sommervile, MA 02145 (US); Tramontano, Valentino, Brockton, MA 02301 (US)
(74) Representative: Soames, Candida Jane

(57) **Abstract**

Processes for coating medical devices are provided herein. The processes include heating a surface of the particles used to form the coating as the particles are being applied to the medical device. The resulting coating has improved adherence to the medical device, and does not require the use of solvents and/or water, obviating the need for any steps that otherwise might be required to remove these solvents and/or water. Sufficient adherence of the particles to the medical device may also occur without the need for heating the substrate used to form the medical device.

## Description

### TECHNICAL FIELD

The present disclosure relates to coated implants. More particularly, the present disclosure relates to methods for coating medical implants, in embodiments surgical meshes, by heating of the particles used to form the coating as they are being applied to the implant.

### BACKGROUND

Techniques for repairing damaged or diseased tissue are widespread in medicine. Wound closure devices, such as sutures and staples, as well as other repair devices like mesh or patch reinforcements, are frequently used for repair.

Coatings have been applied to medical devices to impart lubricious and/or anti-adhesive properties and serve as depots for bioactive agent release. Adherence of these coatings to the substrate used to form the device may prove difficult, with delamination occurring in some cases. In addition, some processes use materials, such as solvents, which may require additional steps for their removal, thereby increasing the costs associated with forming the medical device.

Improved coatings for medical devices, and processes for their application, thus remain desirable.

### SUMMARY

The present disclosure provides methods for applying coatings to medical devices, as well as medical devices possessing such coatings. In embodiments, a method of the present disclosure includes providing a medical device including a substrate; providing a source of polymeric particles; applying the polymeric particles to a surface of the substrate; and heating a surface of the particles as they travel from the source of the particles to the substrate, wherein the particles form a coating on at least a portion of the surface of the substrate upon contact therewith.

In other embodiments, a method of the present disclosure includes providing a medical device including a substrate; providing a source of polymeric particles; applying the polymeric particles to a surface of the substrate, the polymeric particles including at least one monomer such as glycolide, lactide, p-dioxanone, ε-caprolactone, trimethylene carbonate, orthoesters, phosphoesters, and combinations thereof; and heating a surface of the particles to a temperature above the glass transition temperature of the polymeric particles as they travel from the source of the particles to the substrate, wherein the particles form a coating on at least a portion of the surface of the substrate upon contact therewith.

Systems for applying these coatings to medical devices are also provided. In embodiments, a system of the present disclosure includes at least one source of polymeric particles; at least one substrate; at least one spraying unit for applying the polymeric particles to the substrate; and at least one heating unit for heating a surface of the particles as they travel from the source of polymeric particles to the substrate.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

Figure 1 illustrates a system of the present disclosure for applying a coating to a medial device;

Figure 2 illustrates an alternate system of the present disclosure for applying a coating to a medial device;

Figure 3 illustrates an alternate system of the present disclosure for applying a coating to a medial device;

Figure 4 illustrates an alternate system of the present disclosure for applying a coating to a medial device;

Figure 5 illustrates an alternate system of the present disclosure for applying a coating to a medial device;

Figure 6 illustrates an alternate system of the present disclosure for applying a coating to a medial device;

Figure 7 is a graph of heat flow (in W/g) versus temperature, as obtained by Differential Scanning Calorimetry (DSC), for a poly-lactide-co-glycolide copolymer (PLGA);

Figure 8 is a graph of heat flow (in W/g) versus temperature, as obtained by DSC, for bupivacaine;

Figure 9 is a graph of heat flow (in W/g) versus temperature, as obtained by DSC, for three formulations of bupivacaine loaded micro-particles of the present disclosure;

Figure 10 combines the DSC curves for the PLGA co-polymer, the bupivacaine, and one of the formulations of the bupivacaine loaded micro-particles of the present disclosure;

Figure 11 is a graph showing weight versus temperature for formulation A of the bupivacaine loaded micro-particles of the present disclosure, as determined by Thermal Gravimetric Analysis;

Figure 12 is a graph showing weight versus temperature for formulation B of the bupivacaine loaded micro-particles of the present disclosure, as determined by Thermal Gravimetric Analysis; and

Figure 13 is a graph showing weight versus temperature for formulation C of the bupivacaine loaded micro-particles of the present disclosure, as determined by Thermal Gravimetric Analysis.

### DETAILED DESCRIPTION

The processes of the present disclosure may be used, in embodiments, to apply coatings to medical devices. Substrates used to form medical devices in accordance with the present disclosure may be formed of any suitable substance, including metals, polymers, ceramics, combinations thereof, and the like.

The medical devices of the present disclosure include a surface coating formed from particles. The particles may be nanoparticles, microparticles, combinations thereof, and the like. For example, in embodiments, the particles may be nanoparticles having an average particle diameter from about 50 nm to about 1000 nm, in embodiments from about 200 nm to about 800 nm, in other embodiments from about 300 nm to about 600 nm. In other embodiments, the particles may be microparticles possessing an average particle diameter of from about 5 microns (µ) to about 180 µ, in embodiments from about 25 µ to about 150 µ, and in embodiments from about 45 µ to about 105 µ. Other sized particles may be used, in embodiments.

In embodiments, the particles may be formed of polymers. Polymers which may be used to form particles suitable for use in forming a coating for a medical device include, in embodiments, biodegradable polymers. Suitable biodegradable materials which may be utilized to form the polymeric coatings in accordance with the present disclosure include homopolymers, copolymers, and/or blends possessing glycolide, lactide, p-dioxanone, ε-caprolactone, trimethylene carbonate, orthoesters, phosphoesters, polysaccharides, modified starches, cellulose, oxidized cellulose, and various combinations of the foregoing. Methods for forming such copolymers are within the purview of those skilled in the art and include, for example, the methods disclosed in U.S. Patent Nos. 4,300,565 and 5,324,307, the entire disclosures of each of which are incorporated by reference herein.

In embodiments, glycolide and lactide based polyesters may be utilized. These polymers include, for example, poly-lactide-co-glycolide (PLGA) copolymers. Suitable copolymers of lactide and glycolide may possess lactide in amounts from about 50% to about 99% by weight of the copolymer, in embodiments, from about 60% to about 85% by weight of the copolymer, with the glycolide being present in amounts from about 1% to about 50% by weight of the copolymer, in embodiments, from about 15% to about 40% by weight of the copolymer.

In some embodiments, the surface coating may contain additional components. Such additional components include conventional additives capable of providing desirable characteristics to a coating, such as dyes, bioactive agents, lubricants, adhesives, including carboxy methyl cellulose (CMC), fatty acid components, polymeric components, PEG substituted succinimides and glutamides, combinations thereof, and the like. In embodiments, a coating may include a fatty acid component, such as a fatty acid or a fatty acid salt or a salt of a fatty acid ester. For example, a polyethylene glycol fatty acid ester, such as PEG monostearate, PEG monooleate, PEG distearate, PEG diisostearate, PEG stearates, and PEG triglycerides may be utilized as a component of the surface coating. In other embodiments, a coating of the present disclosure may include at least one bioactive agent.

In embodiments, a PEG cross-linker may be used in forming the particles. Such a PEG cross-linker may, in embodiments, be a therapeutic agent. Examples of such cross-linkers, as well as matrices formed therewith, include those disclosed in U.S. Patent Application Serial No.: 13/017,287, filed January 31, 2011, the entire disclosure of which is incorporated by reference herein.

In embodiments, a surface coating of the present disclosure may include from about 90% to about 99% of the biodegradable polymer, e.g., a lactide/glycolide copolymer, with the additive component being present in an amount from about 1% to about 10% of the surface coating. In embodiments, the surface coating may include from about 95% to about 99% of the biodegradable polymer with the additive component being present in an amount from about 1% to about 5% of the surface coating, and in some embodiments, the surface coating may include from about 97% to about 99% of the biodegradable polymer with the additive component being present in an amount from about 1% to about 3% of the surface coating.

Particles may be formed using any method within the purview of those skilled in the art. Suitable methods for the formation of particles include spray-drying, solvent evaporation, and phase separation. For spray drying, a polymer may be mixed with a solvent for the polymer, and then the solvent is evaporated by spraying the solution, leaving polymeric droplets. Solvent evaporation involves dissolving the polymer in an organic solvent, which is then added to an agitated continuous phase (which is often aqueous). Emulsifiers are included in the aqueous phase to stabilize the oil-in-water emulsion. The organic solvent is then extracted over a period of several hours or more, leaving behind the polymer in particluate form. Phase separation involves the formation of a water-in-oil emulsion or an oil-in-water emulsion; however, other forms of emulsions may be used, including oil-in-oil, water-in-oil-in-water, oil-in-water-in-oil, or oil-in-oil-in-oil emulsions. The polymer is precipitated from the continuous phase by a change in temperature, pH, ionic strength, or the addition of precipitants. For a review of phase separation techniques, see e.g. U.S. Pat. No. 4,675,800 (and references cited therein). Other suitable processes for forming micro-particles include those disclosed in U.S. Patent Nos. 6,020,004 and 5,858,531, the disclosures of each of which are incorporated by reference herein.

In embodiments, the particles may encapsulate any additive, such as a bioactive agent, or a combination of bioactive agents.

After formation, the particles are applied to the substrate used to form the medical device without the use of solvents and/or water, i.e., by spray coating, including air-assisted spraying, air-atomized spraying, ultrasonic spraying, electrospraying, airless spraying, and/or high volume, low pressure spraying; powder coating; combinations thereof, and the like. A surface of the particles is heated during their flight from a dispensing source to the substrate surface, to a temperature above the glass transition and/or melting temperature of the polymer(s) used to form the particles. The heated surface of the particles thus has enhanced adherence to the substrate to which the particles are applied, thereby forming an adherent coating upon contact with the substrate.

In embodiments, the particles may be sprayed onto a surface of a substrate via any conventional spraying device, including a spray nozzle, atomizer, nebulizer, combinations thereof, and the like.

Sources of heat which may be utilized to heat a surface of the particles in flight include any heat source capable of heating the surface of the particles to a temperature above the glass transition and/or melting temperature of any polymer(s) used to form the particles. Such heat sources include electromagnetic radiation, for example, infrared (IR), ultrasound, microwave, radiofrequency (RF), visible light, combinations thereof, and the like. In addition, the heat source may initiate an exothermic reaction on the surface of the micro-particle, which then heats a surface of the micro-particle to a temperature above the glass transition and/or melting temperature of any polymer(s) used to form the particles.

As noted above, in embodiments a polymer used to form the particles may be a PLGA copolymer. Such copolymers may have a glass transition temperature (Tg) of from about 35°C to about 65°C; however, with the inclusion of additives, the Tg of the loaded particles can be from about 35°C to about 200°C, and thus it may be desirable to heat a surface of the particles in flight to a temperature of from about 35°C to about 120°C, in embodiments from about 40°C to about 100°C, and in embodiments from about 50°C to about 85°C. Upon contact with the substrate, the particles form a coating thereon, with enhanced adherence to the substrate.

Embodiments of the presently disclosed system and methods will now be described in detail with reference to the drawing figures, wherein like reference numerals identify similar or identical elements.

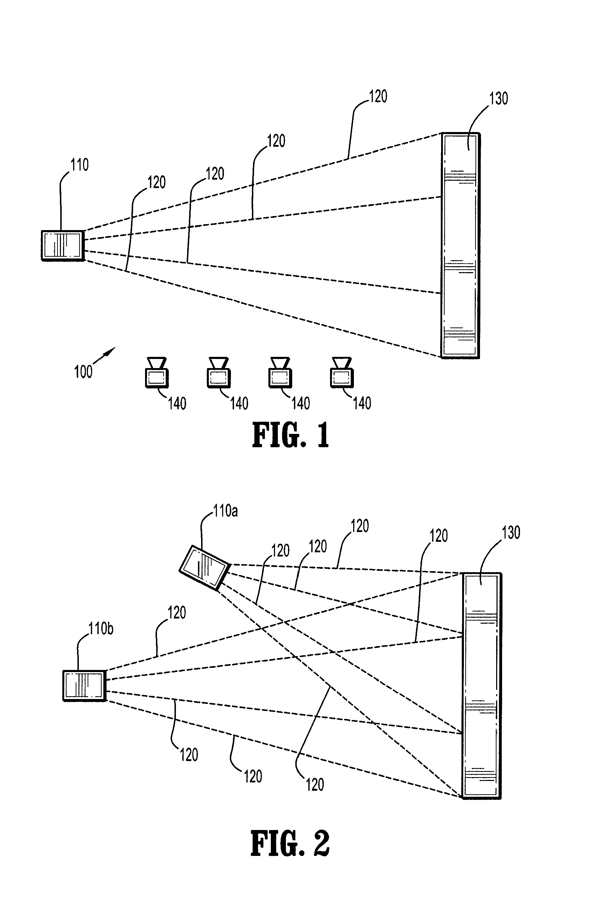

Turning first to Figure 1, an exemplary system 100 for applying particles 120 in accordance with the present disclosure is depicted therein. Spraying unit 110 directs particles 120 at substrate 130. Heating units 140 are placed adjacent the flight path of particles 120 from spraying unit 110 to substrate 130. Heating units 140 may be any suitable source of heat capable of heating a surface of the particles to a temperature above the glass transition and/or melting temperature of the polymer(s) used to form the particles. While the Figure shows four heating units 140, any suitable number of heating sources may be utilized in any configuration, so long as a surface of the polymeric particles is sufficiently heated as the particles travel from their source to the substrate for application thereto.

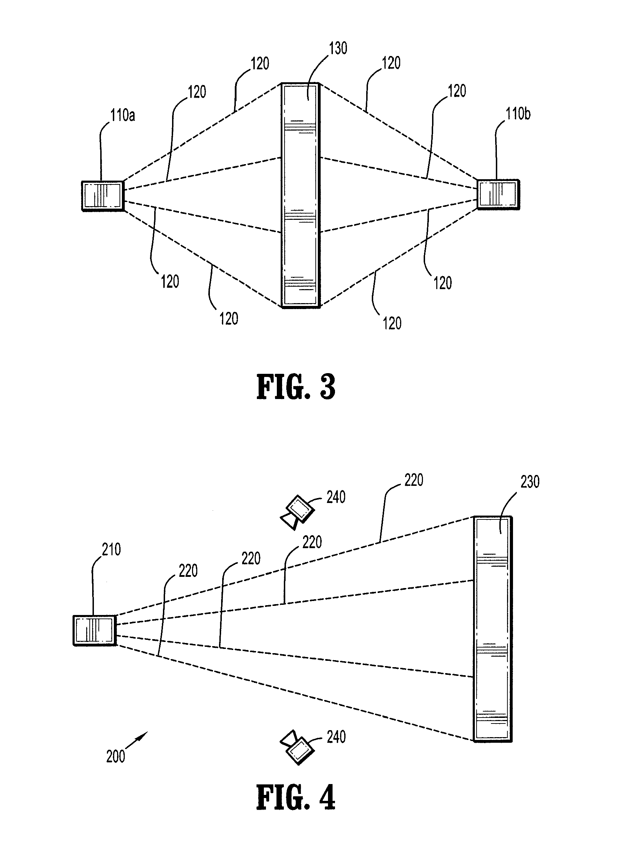

In addition, as depicted in Figures 2 and 3, in embodiments, multiple spraying units 110a and 110b may direct particles 120 to substrate 130. As depicted in Figure 2, spraying units 110a and 110b may direct particles 120 at different angles to substrate 130 or, as depicted in Figure 3, spraying units 110a and 110b may direct particles 120 at opposite sides of substrate 130.

An alternate system 200 for applying particles 220 is set forth in Figure 4. Spraying unit 210 directs particles 220 at substrate 230. Heating units 240 are placed adjacent the flight path of particles 220 from spraying unit 210 to substrate 230. As seen in Figure 4, heating units 240 may be placed at an angle to provide directional heating of the surface of the particles 220, so that the surfaces of particles 220 that will contact substrate 230 are heated. Heating units 240 may be any suitable source of heat capable of heating a surface of the particles to a temperature above the glass transition and/or melting temperature of the polymer(s) used to form the particles. While Figure 4 shows two heating units 240, any suitable number of heating sources may be utilized.

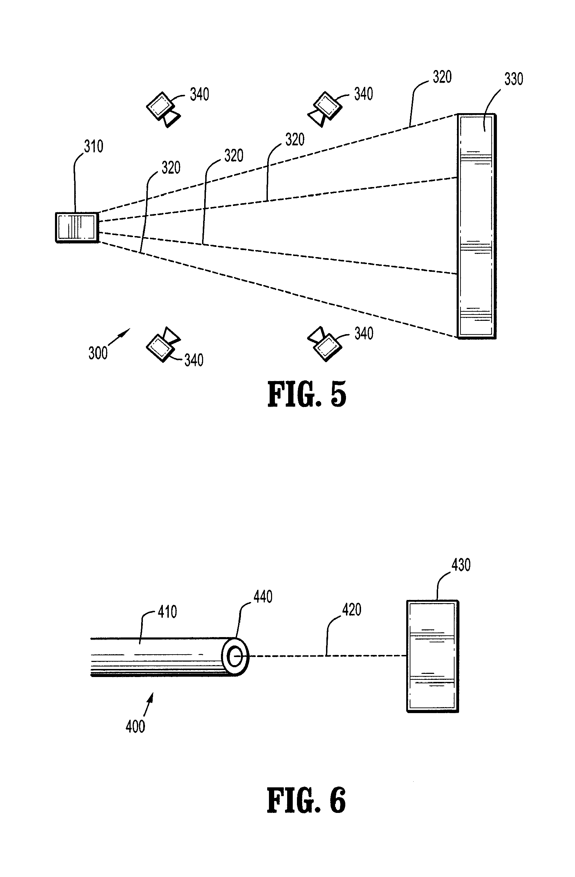

An alternate system 300 for applying particles 320 is set forth in Figure 5. Spraying unit 310 directs particles 320 at substrate 330. Heating units 340 are placed adjacent the flight path of particles 320 from spraying unit 310 to substrate 330. As seen in Figure 5, heating units 340 may be placed at varying angles to provide directional heating of both the front and back surfaces of the particles 320, thereby providing uniform heating of the surface of particles 320. Heating units 340 may be any suitable source of heat capable of heating a surface of the particles to a temperature above the glass transition and/or melting temperature of the polymer(s) used to form the particles. While Figure 5 shows four heating units 340, any suitable number of heating sources may be utilized. For example, additional heating units (not shown) may be placed at additional angles, thereby providing for additional multi-directional heating of the surfaces of particles 320. Additionally, the heating units 340 may be of the same or different types of heat sources.

Yet another alternate system 400 for applying particles 420 is set forth in Figure 6. Spraying unit 410 directs particles 420 at substrate 430. Spraying unit 410 may possess heating unit 440 as a part thereof. As seeen in Figure 6, heating unit 440, which is depicted as a ring adjacent the mouth of spraying unit 410 where the particles 420 are ejected towards substrate 430, heats particles 420 as they are ejected from spraying unit 410. Heating unit 440 may be any suitable source of heat capable of heating a surface of the particles to a temperature above the glass transition and/or melting temperature of the polymer(s) used to form the particles. While Figure 6 shows a single heating unit 440 in the form of a ring, alternate configurations are envisioned. For example, although not shown, in embodiments the heating unit could be a tube extending along the external surface of spraying unit 410, or multiple heating units configured as rings could be placed along the external surface of spraying unit 410, to provide additional heating of the particles as they travel through and/or out of spraying unit 410.

Moreover, while not shown, combinations of the above systems could be utilized. For example, a spraying unit possessing a heat source, as depicted in Figure 6, could be utilized with additional heating unit configurations as depicted in any of Figures 1-6.

The processes of the present disclosure have several advantages for application of coatings to medical devices. As a surface of the particles utilized to form the coating is heated in flight, heating of the substrate to which the particles are to be applied is not required, which can minimize and/or prevent any damage or degradation to the substrate that might otherwise occur if the substrate itself was heated. For example, substrates formed of nylon, caprolactone, propylene, ethylene, polyethylene terephthalate, combinations thereof, and the like, might be damaged by heating. Similarly, substrates formed of metals and composite materials may undergo chemical and/or oxidative change upon heating, which could negatively affect the surface characteristics of the device. Additionally, medical devices are often coated to enhance the handling or performance characteristic of the device, and the performace of such coatings, e.g., an antibiotic coating, may be negatively affected by heating. The processes of the present disclosure avoid such damage, as the substrate is not heated. Moreover, as the substrate is not heated, it may instead be cooled, which allows the substrate to quench the micro-particle upon the particle's impact upon the substrate. This quenching may thereby enhance the micro-particle's adhesion to the substrate.

Moreover, as noted above, the processes of the present disclsoure avoid the use of solvents and aqueous media, simplifying the processes of application, which do not require separate drying steps for the removal of solvents and/or water.

In some cases, the coating may be annealed after application of the particles. In other embodiments, the substrate may be kept at room temperature and/or cooled as the particles are applied thereto. In this manner, the heated surfaces of the particles adhere to the surface of the substrate, forming a coating thereon, with annealing of the coating occurring almost immediately, as the cooler substrate anneals the applied coating. (Such annealing might not be practical if the substrate had to be heated for application of the coating.) Moreover, certain substrates, such as metals, may be readily cooled and thus a coating applied to a metal substrate in accordance with the present disclosure may be readily annealed by cooling a metal surface while applying particles thereto in accordance with the present disclosure.

In embodiments, most of the accessible surfaces of the substrate may be covered with the particles. In yet other embodiments, the entire substrate is covered. The coating may cover from about 1% to about 100% of the area of the substrate, in embodiments a mesh, in embodiments from about 20% to about 80% of the area of the substrate, and in embodiments from about 40% to about 70% of the area of the substrate. The amount of coating may also be by weight percent of the coated substrate, i.e., the coating may be present in an amount of from about 0.001% to about 50% by weight of the total weight of the substrate, in embodiments, from about 0.01% to about 10% by weight of the total weight of the substrate, and in embodiments, from about 0.1% to about 5% by weight of the total weight of the substrate.

Suitable medical devices which may be coated in accordance with the present disclosure include, but are not limited to, clips and other fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, contact lenses, intraocular lenses, anti-adhesion devices, anchors, tunnels, bone fillers, synthetic tendons, synthetic ligaments, tissue scaffolds, stapling devices, buttresses, lapbands, orthopedic hardware, pacers, pacemakers, and other implants and implantable devices.

Fibers can be made from, or coated with, the compositions of the present disclosure. In embodiments, fibers made or coated with the compositions of the present disclosure may be knitted or woven with other fibers, either absorbable or non-absorbable fibers, to form textiles. The fibers also can be made into non-woven materials to form fabrics, such as meshes and felts.

Bioactive agents may be added to a medical device of the present disclosure, either as part of the device, and/or as part of the coating applied in accordance with the present disclosure. A "bioactive agent," as used herein, includes any substance or mixture of substances that provides a therapeutic or prophylactic effect; a compound that affects or participates in tissue growth, cell growth and/or cell differentiation; a compound that may be able to invoke or prevent a biological action such as an immune response; or a compound that could play any other role in one or more biological processes. A variety of bioactive agents may be incorporated into the medical device. Moreover, any agent which may enhance tissue repair, limit the risk of sepsis, and modulate the mechanical properties of the mesh (e.g., the swelling rate in water, tensile strength, etc.) may be added during the preparation of the surgical mesh or may be coated on or into the openings of the mesh. The bioactive agent may be applied to the individual fibers of the surgical mesh or may be applied to the formed surgical mesh, or just one or more sides or portions thereof. In embodiments, the bioactive agent may be added to the surface coating.

Examples of classes of bioactive agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Other bioactive agents which may be in the present disclosure include: local anesthetics such as bupivacaine; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists such as naltrexone and naloxone; anticancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included in the present disclosure include: viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, (α-IFN and γ-IFN)); erythropoietin; nucleases; tumor necrosis factor; colony stimulating factors (e.g., GCSF, GM-CSF, MCSF); insulin; anti-tumor agents and tumor suppressors; blood proteins; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); protein inhibitors; protein antagonists; protein agonists; nucleic acids such as antisense molecules, DNA, and RNA; oligonucleotides; and ribozymes.

As noted above, in embodiments, a medical device coated by the process of the present disclsoure may be a surgical mesh. The meshes of the present disclosure can be in the form of sheets, patches, slings, suspenders, and other implants and composite materials such as pledgets, buttresses, wound dressings, drug delivery devices, and the like. The present surgical meshes may be implanted using open surgery or by a laparoscopic procedure.

A surgical mesh in accordance with the present disclosure may be fabricated from monofilament and/or multifilament yarns which may be made of any suitable biocompatible material. Suitable materials from which the mesh can be made should have the following characteristics: sufficient tensile strength to support tissue; sufficiently inert to avoid foreign body reactions when retained in the body for long periods of time; easily sterilized to prevent the introduction of infection when the mesh is implanted in the body; and sufficiently strong to avoid tearing of portions thereof, including any portion through which surgical fasteners may be applied to affix the mesh to tissue.

In some embodiments, the yarns include at least two filaments which may be arranged to create openings therebetween, the yarns also being arranged relative to each other to form openings in the mesh. Alternatively, the mesh may be formed from a continuous yarn that is arranged in loops that give rise to the openings in the mesh. The use of a mesh having yarns spaced apart in accordance with the present disclosure has the advantage of reducing the foreign body mass that is implanted in the body, while maintaining sufficient tensile strength to securely support the defect and tissue being repaired by the mesh. Moreover, the openings of the mesh of the present disclosure may be sized to permit fibroblast through-growth and ordered collagen laydown, resulting in integration of the mesh into the body. Thus, the spacing between the yarns may vary depending on the surgical application and desired implant characteristics as envisioned by those skilled in the art. Moreover, due to the variety of sizes of defects, and of the various fascia that may need repair, the mesh may be of any suitable size.

In embodiments in which at least two filaments form a yarn, the filaments may be drawn, oriented, crinkled, twisted, braided, commingled or air entangled to form the yarn. The resulting yarns may be braided, twisted, aligned, fused, or otherwise joined to form a variety of different mesh shapes. The yarns may be woven, knitted, interlaced, braided, or formed into a surgical mesh by non-woven techniques. The structure of the mesh will vary depending upon the assembling technique utilized to form the mesh, as well as other factors, such as the type of fibers used, the tension at which the yarns are held, and the mechanical properties required of the mesh.

In embodiments, knitting may be utilized to form a mesh of the present disclosure. Knitting involves, in embodiments, the intermeshing of yarns to form loops or inter-looping of the yarns. In embodiments, yarns may be warp-knitted thereby creating vertical interlocking loop chains, and/or yarns may be weft-knitted thereby creating rows of interlocking loop stitches across the mesh. In other embodiments, weaving may be utilized to form a mesh of the present disclosure. Weaving may include, in embodiments, the intersection of two sets of straight yarns, warp and weft, which cross and interweave at right angles to each other, or the interlacing of two yarns at right angles to each other. In some embodiments, the yarns may be arranged to form a net mesh which has isotropic or near isotropic tensile strength and elasticity.

In embodiments, the yarns may be nonwoven and formed by mechanically, chemically, or thermally bonding the yarns into a sheet or web in a random or systematic arrangement. For example, yarns may be mechanically bound by entangling the yarns to form the mesh by means other than knitting or weaving, such as matting, pressing, stitch-bonding, needlepunching, or otherwise interlocking the yarns to form a binderless network. In other embodiments, the yarns of the mesh may be chemically bound by use of an adhesive such as a hot melt adhesive, or thermally bound by applying a binder such as a powder, paste, or melt, and melting the binder on the sheet or web of yarns.

The yarns may be fabricated from any biodegradable and/or non-biodegradable polymer that can be used in surgical procedures. The term "biodegradable" as used herein is defined to include both bioabsorbable and bioresorbable materials. By biodegradable, it is meant that the material decomposes, or loses structural integrity under body conditions (e.g., enzymatic degradation or hydrolysis) or is broken down (physically or chemically) under physiologic conditions in the body, such that the degradation products are excretable or absorbable by the body. Absorbable materials are absorbed by biological tissues and disappear *in vivo* at the end of a given period, which can vary, for example, from hours to several months, depending on the chemical nature of the material. It should be understood that such materials include natural, synthetic, bioabsorbable, and/or certain non-absorbable materials, as well as combinations thereof.

Representative natural biodegradable polymers which may be used to form the yarns include: polysaccharides such as alginate, dextran, chitin, chitosan, hyaluronic acid, cellulose, collagen, gelatin, fucans, glycosaminoglycans, and chemical derivatives thereof (substitutions and/or additions of chemical groups including, for example, alkyl, alkylene, amine, sulfate, hydroxylations, carboxylations, oxidations, and other modifications routinely made by those skilled in the art); catgut; silk; linen; cotton; and proteins such as albumin, casein, zein, silk, soybean protein; and combinations such as copolymers and blends thereof, alone or in combination with synthetic polymers.

Synthetically modified natural polymers which may be used to form the yarns include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, and combinations thereof.

Representative synthetic biodegradable polymers which may be utilized to form yarns include polyhydroxy acids prepared from lactone monomers (such as glycolide, lactide, caprolactone, ε-caprolactone, valerolactone, and δ-valerolactone), carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s such as polyhydroxybutyrate, polyhydroxyvalerate, poly(3-hydroxybutyrate-co-3-hydroxyvalerate), polyhydroxyoctanoate, and polyhydroxyhexanoate; polyalkylene oxalates; polyoxaesters; polyanhydrides; polyester anyhydrides; polyortho esters; and copolymers, block copolymers, homopolymers, blends, and combinations thereof.

Synthetic degradable polymers also include hydrophilic vinyl polymers expanded to include phosphoryl choline such as 2-methacryloyloxyethyl phosphorylcholine, hydroxamates, vinyl furanones and their copolymers, and quaternary ammonia; as well as various alkylene oxide copolymers in combination with other polymers such as lactones, orthoesters, and hydroxybutyrates, for example.

Rapidly bioerodible polymers, such as poly(lactide-co-glycolide)s, polyanhydrides, and polyorthoesters, which have carboxylic groups exposed on the external surface as the surface of the polymer erodes, may also be used.

Other biodegradable polymers include polyphosphazenes; polypropylene fumarates; polyimides; polymer drugs such as polyamines; perfluoroalkoxy polymers; fluorinated ethylene/propylene copolymers; PEG-lactone copolymers; PEG-polyorthoester copolymers; blends and combinations thereof.

Some non-limiting examples of suitable nondegradable materials from which the mesh may be made include polyolefins such as polyethylene (including ultra high molecular weight polyethylene) and polypropylene including atactic, isotactic, syndiotactic, and blends thereof; polyethylene glycols; polyethylene oxides; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; etheylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof.

The mesh may be a composite of layers, including a fibrous layer as described above, as well as porous and/or non-porous layers of fibers, foams, and/or films. A non-porous layer may retard or prevent tissue ingrowth from surrounding tissues, thereby acting as an adhesion barrier and preventing the formation of unwanted scar tissue. In embodiments, a reinforcement member may be included in the composite mesh. Suitable meshes, for example, include a collagen composite mesh such as PARIETEX^{™} (Tyco Healthcare Group LP, d/b/a Covidien, North Haven, CT). PARIETEX^{™} composite mesh is a 3-dimensional polyester weave with a resorbable collagen film bonded on one side. Examples of other meshes which may be utilized include those disclosed in U.S. Patent Nos. 6,596,002; 6,408,656; 7,021,086; 6,971,252; 6,695,855; 6,451,032 ; 6,443,964; 6,478,727; 6,391,060; and U.S. Patent Application Publication No. 2007/0032805, the entire disclosures of each of which are incorporated by reference herein.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20 °C to about 30° C.

### EXAMPLES

### EXAMPLE 1

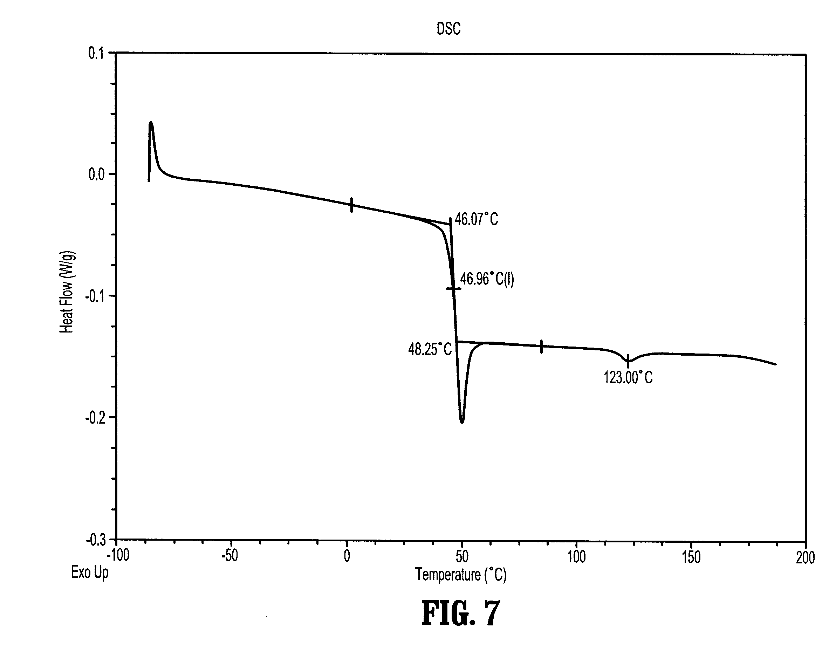

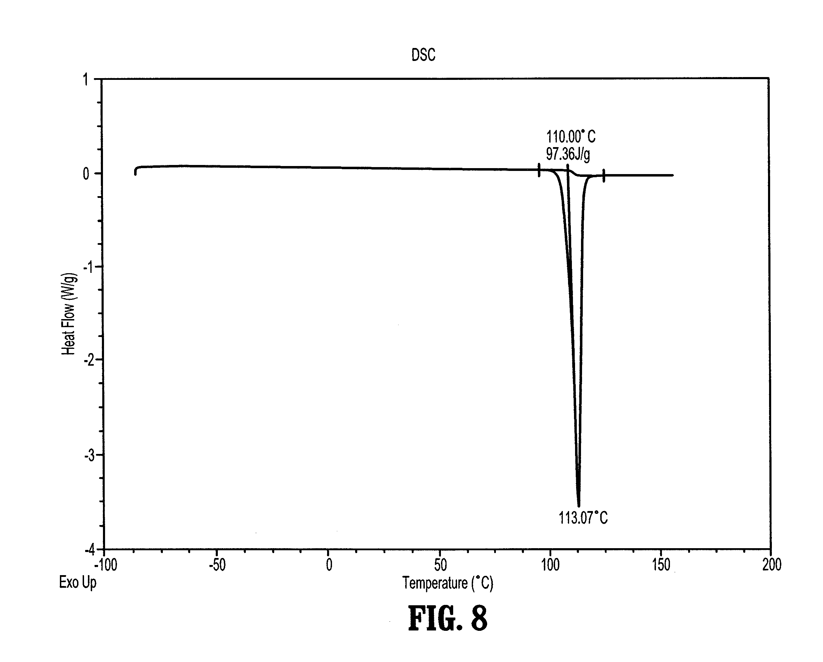

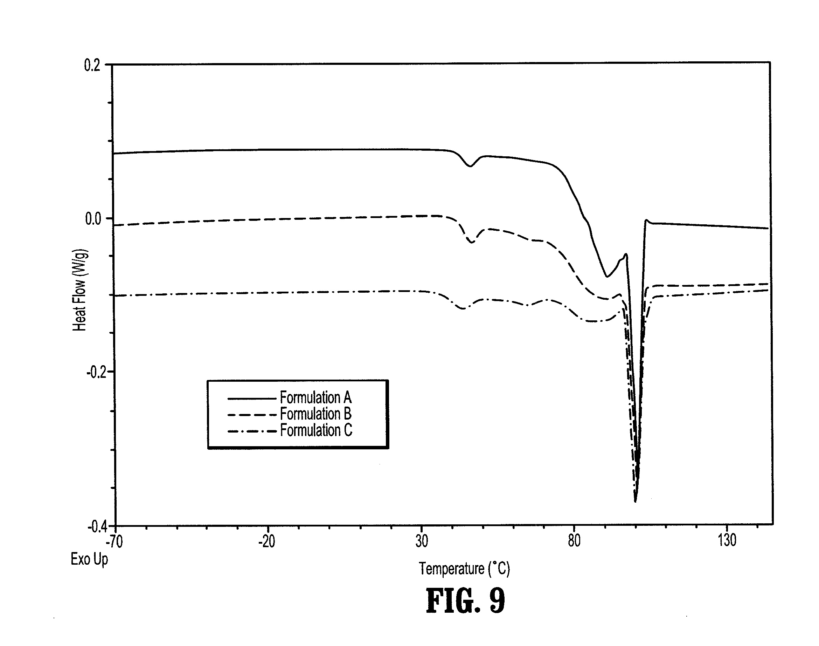

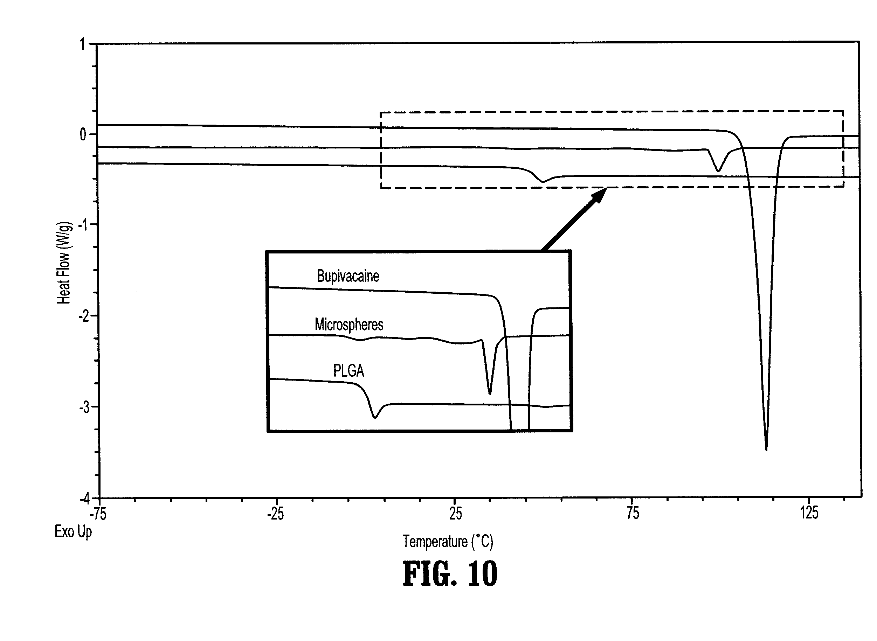

Differential Scanning Calorimetry was performed on: (1) a poly-lactide-co-glycolide (PLGA) copolymer, including about 75% lactide and about 25% glycolide; (2) bupivacaine; and (3) three formulations of the present disclosure, including the bupivacaine loaded into micro-particles formed of the PLGA copolymer. The three formulations were designed "A," "B," and "C." The results are set forth in Figures 7, 8, 9, and 10. As can be seen from Figure 7, the glass transition temperature of the PLGA co-polymer was about 47°C. As can be seen from Figure 8, the glass transition temperature of bupivacaine was about 112.5°C. Finally, as can be seen from Figure 9, the glass transition temperature of the three formulations of the bupivacaine loaded PLGA micro-particles showed some phase transtion at just under 50°C, and a glass transition temperature of about 100°C. Figure 10 combines the DSC curves for the PLGA co-polymer, the bupivacaine, and one of the formulations of the bupivacaine loaded PLGA micro-particles (formulation C).

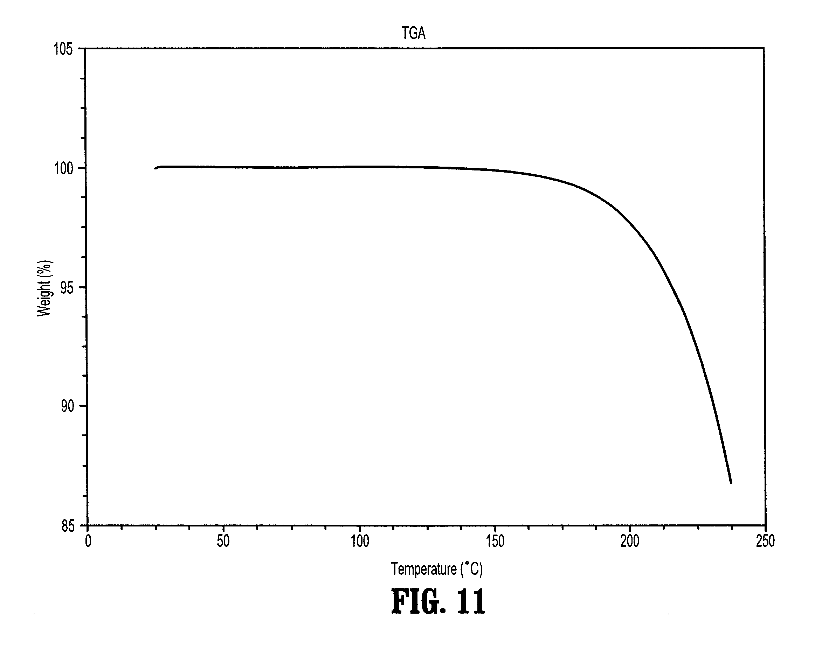

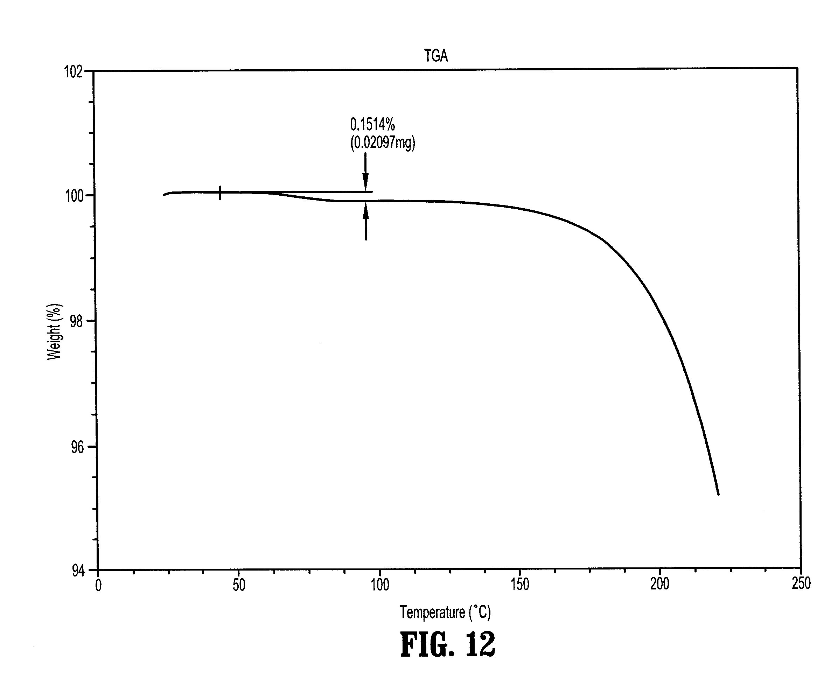

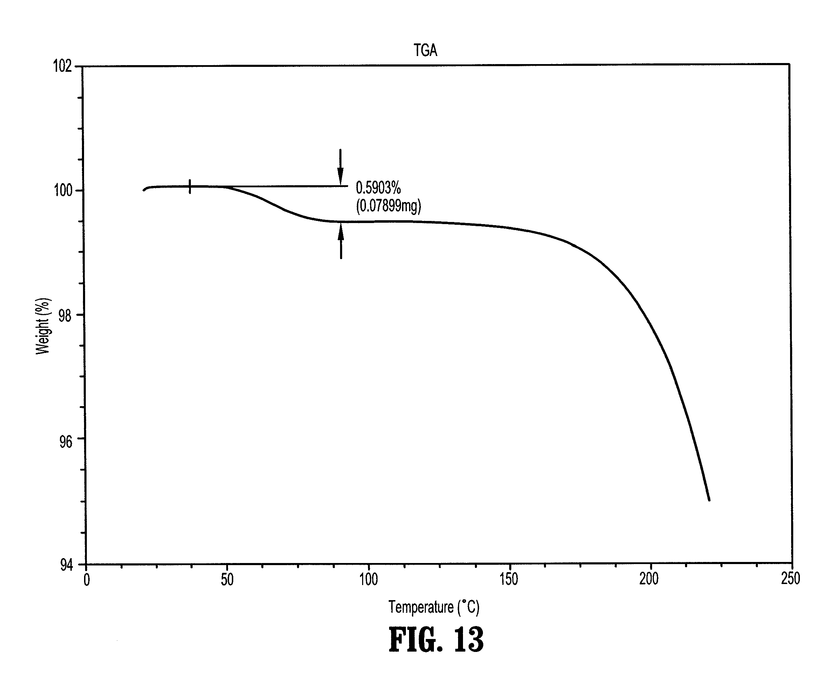

Thermal Gravimetric Analysis was conducted on the three formulations of the bupivacaine loaded PLGA micro-particles. The results are set forth in Figures 11, 12 and 13, which show the glass transition temperature (Tg) for formulations A, B, and C, respectively. As can be seen from Figures 11-13, the bupivacaine loaded PLGA micro-particles all possessed similar degradation profiles, with each formulation undergoing degradation at temperatures above 150°C.

### EXAMPLE 2

Micro-particles are applied to a surface of a medical device as follows. Micro-particles of a poly-lactide-co-glycolide (PLGA) copolymer, encapsulating a bioactive agent such as bupivacaine, are placed into a spraying unit, such as an air-assisted sprayer. A medical device, such as a mesh, is placed at a suitable distance from the spraying unit. The micro-particles are ejected from the spraying unit, so that they travel to a surface of the medical device. As the micro-particles travel from the spraying unit to the medical device, the micro-particles are heated utilizing at least one infrared (IR) heating unit, so that a surface of the micro-particles is at a temperature above the glass transition temperature of the PLGA copolymer, such as from about 40°C to about 95°C. The micro-particles adhere to the surface of the medical device upon contact therewith.

It will be understood that various modifications may be made to the embodiments disclosed herein. Therefore, the above description should not be construed as limiting, but merely as an exemplification of illustrative embodiments. Those skilled in the art will envision other modifications within the scope and spirit of the present disclosure. Such modifications and variations are intended to come within the scope of the following claims.

The invention may be described by reference to the following numbered paragraphs:-
1. A method comprising:
   providing a medical device comprising a substrate;
   providing a source of polymeric particles;
   applying the polymeric particles to a surface of the substrate; and
   heating a surface of the particles as they travel from the source of the particles to the substrate,
   wherein the particles form a coating on at least a portion of the surface of the substrate upon contact therewith.
2. The method of paragraph 1, wherein the particles are applied to the substrate by a process selected from the group consisting of spray coating, air-assisted spraying, air-atomized spraying, ultrasonic spraying; electrospraying, airless spraying, high volume, low pressure spraying, powder coating, and combinations thereof.
3. The method of paragraph 1, wherein the surface of the particles is heated by a means selected from the group consisting of infrared, ultrasound, microwave, radiofrequency, visible light, and combinations thereof.
4. The method of paragraph 1, wherein the particles comprise microparticles possessing an average particle diameter of from about 5 µ to about 180 µ.
5. The method of paragraph 1, wherein the particles comprise nanoparticles having an average particle diameter from about 50 nm to about 1000 nm.
6. The method of paragraph 1, wherein the surface of the particles is heated to a temperature above the glass transition temperature of the polymeric particles.
7. The method of paragraph 1, wherein the polymeric particles comprise glycolide, lactide, p-dioxanone, ε-caprolactone, trimethylene carbonate, orthoesters, phosphoesters, and combinations thereof.
8. The method of paragraph 1, wherein the polymeric particles comprise a copolymer of glycolide and lactide.
9. The method of paragraph 8, wherein glycolide is present in an amount from about 10% to about 50% by weight of the copolymer and lactide is present in an amount from about 50% to about 90% by weight of the copolymer.
10. The method of paragraph 8, wherein the surface of the polymeric particles is heated to a temperature of from about 35°C to about 120°C.
11. The method of paragraph 1, wherein the medical device is selected from the group consisting of clips, fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, contact lenses, intraocular lenses, anti-adhesion devices, anchors, tunnels, bone fillers, synthetic tendons, synthetic ligaments, tissue scaffolds, stapling devices, buttresses, lapbands, orthopedic hardware, pacers, pacemakers, fibers, textiles, and implants.
12. The method of paragraph 1, wherein the medical device comprises a mesh.
13. The method of paragraph 1, further comprising cooling the substrate as the particles are applied thereto.
14. A method comprising:
   providing a medical device comprising a substrate;
   providing a source of polymeric particles;
   applying the polymeric particles to a surface of the substrate, the polymeric particles comprising at least one monomer selected from the group consisting of glycolide, lactide, p-dioxanone, ε-caprolactone, trimethylene carbonate, orthoesters, phosphoesters, and combinations thereof; and
   heating a surface of the particles to a temperature above the glass transition temperature of the polymeric particles as they travel from the source of the particles to the substrate,
   wherein the particles form a coating on at least a portion of the surface of the substrate upon contact therewith.
15. The method of paragraph 14, wherein the particles are applied to the substrate by a process selected from the group consisting of spray coating, air-assisted spraying, air-atomized spraying, ultrasonic spraying; electrospraying, airless spraying, high volume, low pressure spraying, powder coating, and combinations thereof.
16. The method of paragraph 14, wherein the surface of the particles is heated by a means selected from the group consisting of infrared, ultrasound, microwave, radiofrequency, visible light, and combinations thereof.
17. The method of paragraph 14, wherein the particles comprise microparticles possessing an average particle diameter of from about 5 µ to about 180 µ.
18. The method of paragraph 14, wherein the particles comprise nanoparticles having an average particle diameter from about 50 nm to about 1000 nm.
19. The method of paragraph 14, wherein the polymeric particles comprise a copolymer of glycolide and lactide.
20. The method of paragraph 19, wherein glycolide is present in an amount from about 10% to about 50% by weight of the copolymer and lactide is present in an amount from about 50% to about 90% by weight of the copolymer.
21. The method of paragraph 19, wherein the surface of the polymeric particles is heated to a temperature of from about 35°C to about 120°C.
22. The method of paragraph 14, wherein the medical device is selected from the group consisting of clips, fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, contact lenses, intraocular lenses, anti-adhesion devices, anchors, tunnels, bone fillers, synthetic tendons, synthetic ligaments, tissue scaffolds, stapling devices, buttresses, lapbands, orthopedic hardware, pacers, pacemakers, fibers, textiles, and implants.
23. The method of paragraph 14, wherein the medical device comprises a mesh.
24. The method of paragraph 14, further comprising cooling the substrate as the particles are applied thereto.
25. A system for applying a coating to a medical device comprising:
   at least one source of polymeric particles;
   at least one substrate;
   at least one spraying unit for applying the polymeric particles to the substrate; and
   at least one heating unit for heating a surface of the particles as they travel from the source of polymeric particles to the substrate.

## Claims

1. A method comprising:
providing a medical device comprising a substrate;
providing a source of polymeric particles;
applying the polymeric particles to a surface of the substrate; and
heating a surface of the particles as they travel from the source of the particles to the substrate,
wherein the particles form a coating on at least a portion of the surface of the substrate upon contact therewith.

2. The method of claim 1, wherein the particles are applied to the substrate by a process selected from the group consisting of spray coating, air-assisted spraying, air-atomized spraying, ultrasonic spraying; electrospraying, airless spraying, high volume, low pressure spraying, powder coating, and combinations thereof; and/or wherein the surface of the particles is heated by a means selected from the group consisting of infrared, ultrasound, microwave, radiofrequency, visible light, and combinations thereof.

3. The method of claim 1 or claim 2, wherein the particles comprise microparticles possessing an average particle diameter of from about 5 µ to about 180 µ; and/or wherein the particles comprise nanoparticles having an average particle diameter from about 50 nm to about 1000 nm.

4. The method of any preceding claim, wherein the surface of the particles is heated to a temperature above the glass transition temperature of the polymeric particles.

5. The method of any preceding claim, wherein the polymeric particles comprise glycolide, lactide, p-dioxanone, ε-caprolactone, trimethylene carbonate, orthoesters, phosphoesters, and combinations thereof; preferably wherein the polymeric particles comprise a copolymer of glycolide and lactide; preferably still wherein glycolide is present in an amount from about 10% to about 50% by weight of the copolymer and lactide is present in an amount from about 50% to about 90% by weight of the copolymer.

6. The method of claim 5, wherein the surface of the polymeric particles is heated to a temperature of from about 35°C to about 120°C.

7. The method of any preceding claim, wherein the medical device is selected from the group consisting of clips, fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, contact lenses, intraocular lenses, anti-adhesion devices, anchors, tunnels, bone fillers, synthetic tendons, synthetic ligaments, tissue scaffolds, stapling devices, buttresses, lapbands, orthopedic hardware, pacers, pacemakers, fibers, textiles, and implants; preferably wherein the medical device comprises a mesh.

8. The method of any preceding claim, further comprising cooling the substrate as the particles are applied thereto.

9. A method comprising:
providing a medical device comprising a substrate;
providing a source of polymeric particles;
applying the polymeric particles to a surface of the substrate, the polymeric particles comprising at least one monomer selected from the group consisting of glycolide, lactide, p-dioxanone, ε-caprolactone, trimethylene carbonate, orthoesters, phosphoesters, and combinations thereof; and
heating a surface of the particles to a temperature above the glass transition temperature of the polymeric particles as they travel from the source of the particles to the substrate,
wherein the particles form a coating on at least a portion of the surface of the substrate upon contact therewith.

10. The method of claim 9, wherein the particles are applied to the substrate by a process selected from the group consisting of spray coating, air-assisted spraying, air-atomized spraying, ultrasonic spraying; electrospraying, airless spraying, high volume, low pressure spraying, powder coating, and combinations thereof; and/or wherein the surface of the particles is heated by a means selected from the group consisting of infrared, ultrasound, microwave, radiofrequency, visible light, and combinations thereof.

11. The method of claim 10, wherein the particles comprise microparticles possessing an average particle diameter of from about 5 µ to about 180 µ; and/or wherein the particles comprise nanoparticles having an average particle diameter from about 50 nm to about 1000 nm.

12. The method of any of claims 9 to 11, wherein the polymeric particles comprise a copolymer of glycolide and lactide; preferably wherein glycolide is present in an amount from about 10% to about 50% by weight of the copolymer and lactide is present in an amount from about 50% to about 90% by weight of the copolymer.

13. The method of claim 12, wherein the surface of the polymeric particles is heated to a temperature of from about 35°C to about 120°C.

14. The method of any of claims 9 to 13, wherein the medical device is selected from the group consisting of clips, fasteners, staples, sutures, pins, screws, prosthetic devices, wound dressings, bandages, drug delivery devices, anastomosis rings, surgical blades, contact lenses, intraocular lenses, surgical meshes, stents, stent coatings, grafts, catheters, stent/grafts, knotless wound closures, sealants, adhesives, contact lenses, intraocular lenses, anti-adhesion devices, anchors, tunnels, bone fillers, synthetic tendons, synthetic ligaments, tissue scaffolds, stapling devices, buttresses, lapbands, orthopedic hardware, pacers, pacemakers, fibers, textiles, and implants; preferably wherein the medical device comprises a mesh.

15. The method of any of claims 9 to 14, further comprising cooling the substrate as the particles are applied thereto.

16. A system for applying a coating to a medical device comprising:
at least one source of polymeric particles;
at least one substrate;
at least one spraying unit for applying the polymeric particles to the substrate; and
at least one heating unit for heating a surface of the particles as they travel from the source of polymeric particles to the substrate.
